# EUROPÄISCHE PATENTSCHRIFT

(11) **EP 1 085 017 B1**
(45) Veröffentlichungstag und Bekanntmachung des Hinweises auf die Patenterteilung: **11.05.2005**
(21) Anmeldenummer: 00118709.5
(22) Anmeldetag: 30.08.2000
(51) Int. Cl.: C07D 301/12

(54) **Verfahren zur Herstellung von Epoxiden aus Olefinen**
Process for the preparation of epoxides from olefins
Procédé de préparation d'époxides à partir d'oléfines

(30) Priorität: 18.09.1999 DE 19944839
(43) Veröffentlichungstag der Anmeldung: 21.03.2001
(73) Patentinhaber: Degussa AG, 40474 Düsseldorf (DE); Uhde GmbH, 44141 Dortmund (DE)
(72) Erfinder: Thiele, Georg, Dr., 63450 Hanau (DE)
(74) Vertreter: Sternagel, Fleischer, Godemeyer & Partner Patentanwälte

(56) Entgegenhaltungen:
- EP-A- 0 100 118
- EP-A- 0 230 949
- EP-A- 0 795 537
- WO-A-99/48882
- US-A- 5 412 122
- CLERICI M G ET AL: "EPOXIDATION OF LOWER OLEFINS WITH HYDROGEN PEROXIDE AND TITANIUM SILICALITE" JOURNAL OF CATALYSIS,US,ACADEMIC PRESS, DULUTH, MN, Bd. 140, Nr. 1, 1. März 1993 (1993-03-01), Seiten 71-83, XP000562771 ISSN: 0021-9517

## Beschreibung

Die Erfindung betrifft ein Verfahren zur Herstellung von Epoxiden durch Epoxidierung von olefinischen Verbindungen mit Wasserstoffperoxid in Gegenwart eines Titansilikalits als Katalysator, insbesondere zur Herstellung von Propylenoxid.

### Stand der Technik

Aus der EP-B 0 100 118 ist bekannt, dass sich Olefine mit Wasserstoffperoxid epoxidieren und in Gegenwart von Alkoholen in situ zu Glykolethern umsetzen lassen, wenn als Katalysator ein titanhaltiger Zeolith, allgemein als Titansilikalit bezeichnet, eingesetzt wird. Im Hinblick auf die Herstellung von Epoxiden sind die sauren Eigenschaften dieses Katalysators nachteilig, weil ein Teil des gebildeten Epoxids bereits während der Reaktion unter säurekatalysierter Ringöffnung zum Diol oder in Anwesenheit eines Alkohols als Lösungsmittel zu Diolethern weiterreagiert.

Aus der EP 0 230 949 ist bekannt, dass sich die Epoxid-Ringöffnungsreaktion teilweise unterdrücken läßt, wenn der Katalysator vor und/oder während der Epoxidierungsreaktion mit einem Neutralisierungsmittel neutralisiert wird. In der Patentschrift werden als Neutralisierungsmittel starke Basen wie NaOH und KOH und schwache Basen wie Ammoniak, Alkalicarbonate, Alkalihydrogencarbonate sowie Alkalicarboxylate genannt. Dieses Dokument vermittelt zwar eine Lehre zur Behandlung des Katalysators mit einer Base vor der Epoxidierung, jedoch gibt es keine Anregung, wie der Katalysator während der Epoxidierung neutralisiert werden soll.

M.G. Clerici, P. Ingallina beschreiben in J. Catal. 140 (1993) 71-83 das gattungsgemäße Verfahren sowie den Einfluss von Säuren, Basen und Salzen auf die katalytische Wirksamkeit des Titansilikalit-Katalysators. Hiernach ist bekannt, dass die Wirkung eines Neutralisierungsmittels auf die katalytischen Eigenschaften stark von der Menge des Neutralisierungsmittels abhängt. Während die Verwendung einer kleinen Menge des Neutralisierungsmittels zu einer Steigerung der Selektivität führt, erfolgt bei zu großer Menge eine Hemmung der katalytischen Aktivität für die Epoxidierung bis hin zur vollständigen Blockierung der Aktivität des Katalysators. Es ist ferner bekannt, dass im Reaktionsmedium anwesende Säuren die Reaktionsgeschwindigkeit steigern können. Aus H. Gao, G. Lu, J. Suo, S. Li, Appl. Catal. A 138 (1996) 27-38 ist bekannt, dass diese nachteilige Wirkung des Neutralisierungsmittels schon bei geringen Konzentrationen auftritt und daß Konzentrationen an NaOH bzw. KOH von weniger als 600 ppm bereits zu einem starken Verlust an katalytischer Aktivität führen kann.

Das bekannte Verfahren zur Epoxidierung von Olefinen mit Wasserstoffperoxid und einem Titansilkalitkatalysator unter Zusatz von basischen Substanzen hat den Nachteil, dass bisher keine Möglichkeit bekannt ist, im Einzelfall die für die gewünschte selektivitätsverbessernde und gleichzeitig die Reaktionsgeschwindigkeit nicht oder nur mäßig mindernde Wirkung erforderliche Menge an Neutralisierungsmittel im voraus zu bestimmen. Für die praktische Ausführung der Reaktion hat dies den Nachteil, dass bei der Verwendung eines Neutralisierungsmittels bereits eine geringe Änderung der Qualität der Einsatzstoffe und/oder der Eigenschaften des Katalysators zu einer starken und nicht vorhersehbaren Änderung der Aktivität des Katalysators während der Epoxidierung führen kann. Die zuvor genannten Dokumente geben keine Anregung, wie die Menge der dem System zuzusetzenden Base in engen Grenzen kontrolliert werden kann.

Aufgabe der vorliegenden Erfindung ist es, ein gattungsgemäßes Verfahren aufzuzeigen, womit sich die aufgezeigten Nachteile des vorbekannten Verfahrens überwinden lassen. Das Verfahren sollte sich im diskontinuierlichen und insbesondere im kontinuierlichen Betrieb realisieren lassen. Die Erfindung sollte es auch ermöglichen, das Verfahren so durchzuführen, dass bei möglichst hoher Steigerung der Selektivität sich der Umsatz in voraus bestimmbarer Weise verändert.

### Gegenstand der Erfindung

Es wurde überraschenderweise gefunden, dass sich die Aufgabe dadurch lösen läßt, dass die Zugabe der verwendeten Base zu einem Epoxidationsreaktor pH-überwacht erfolgt und die Menge der Base so gewählt wird, dass für das eingesetzte wasserstoffperoxid oder die eingesetzte Mischung aus Wasserstoffperoxid mit einem oder mehreren Lösungsmitteln ein konstanter, durch einen oder mehrere Testversuche vorherbestimmten pH-Wert resultiert.

Gegenstand der Erfindung ist somit ein Verfahren zur Herstellung von Propylenoxid, umfassend Epoxidierung von Propenmit Wasserstoffperoxid in Gegenwart eines Titansilikalitkatalysators, wobei eine Wasserstoffperoxid enthaltende Lösung und Propen als Einsatzstoffe periodisch oder kontinuierlich in einen Epoxidierungsreaktor eingetragen werden und im Verfahren zusätzlich eine Base eingesetzt wird, das dadurch gekennzeichnet ist, dass man die Base unter pH-Kontrolle im Gemisch mit einem oder mehreren der Einsatzstoffe in den Epoxidierungsreaktor einträgt, die pH-Kontrolle in dem/den die Base enthaltenden Gemisch/en mit einem Einsatzstoff durchführt und einen pH-Wert im Bereich von 4 bis 9.5 einstellt und konstant hält. Da der pH-Wert im Reaktionsgemisch maßgeblich die Selektivität und den Umsatz beeinflusst, wird der optimale pH-Wert zuvor durch einen oder mehrere bei unterschiedlichen pH-Werten unter Einsatz gleicher Einsatzstoffe durchgeführte Epoxidations-Testversuche mit anschließender Bestimmung der Selektivität und des Umsatzes ermittelt.

Durch die Wahl eines geeigneten, konstanten pH-Werts läßt sich die Epoxidselektivität in der Epoxidierung von Olefinen mit Wasserstoffperoxid mit einem Titansilicalitkatalysator in reproduzierbarer Weise verbessern, während gleichzeitig die Aktivität des Katalysators nur wenig und in reproduzierbarer Weise abnimmt. Bei der Einstellung eines konstanten pH-Werts wirken sich Schwankungen in der Qualität der Einsatzstoffe oder der Zusammensetzung des Katalysators weniger stark auf den Reaktionsverlauf aus als bei der Zugabe einer konstanten Menge des Neutralisierungsmittels.

Gemäß einer bevorzugten Ausführungsform wird die Base einer wässrigen oder wässrig-organischen Wasserstoffperoxidlösung zugesetzt und der optimale, aus Vorversuchen, etwa einer pH-abhängigen Testreihe ermittelte pH-Wert in der so erhaltenen Lösung eingestellt und konstant gehalten; im Falle der wässrigen Wasserstoffperoxidlösung liegt der optimale pH-Wert im Bereich von 4 bis 6,5 und im Falle einer organisch-wässrigen Wasserstoffperoxidlösung mit mindestens 50 Gew.-% eines organischen wasserlöslichen Lösungsmittels im Bereich von 5 bis 9.5, wobei sich der pH-Wert auf eine Messung mittels einer Glaselektrode bezieht. Bevorzugt wird eine Einstab-Meßkette aus einer Glaselektrode mit integrierter Ag/AgCl-Vergleichselektrode verwendet.

Das Wasserstoffperoxid wird bei dem erfindungsgemäßen Verfahren in Form einer wässrigen Lösung mit einem Wasserstoffperoxidgehalt von 1 bis 90 Gew.-%, bevorzugt von 10 bis 70 Gew.-% und besonders bevorzugt von 30 bis 50 Gew.-% eingesetzt. Das Wasserstoffperoxid kann in Form der im Handel erhältlichen, stabilisierten Lösungen eingesetzt werden. Ebenso geeignet sind nicht stabilisierte, wässrige Wasserstoffperoxidlösungen, wie sie bei dem Anthrachinonverfahren zur Herstellung von Wasserstoffperoxid erhalten werden. Alternativ hierzu kann Wasserstoffperoxid auch als organisch-wässrige Lösung oder organische Lösung eingesetzt werden. Bevorzugt wird dem Epoxidationsreaktor eine mit einer Base versetzte und pH-kontrollierte wässrige oder wässrig-organische Wasserstoffperoxidlösung zugesetzt.

Als Katalysator eignen sich insbesondere kristalline, titanhaltige Zeolithe der Zusammensetzung (TiO₂)x(SiO₂)1-x mit x von 0.001 bis 0.05 und einer MFI- bzw. MEL-Kristallstruktur, bekannt als Titansilicalit-1 und Titansilicalit-2. Der Titansilicalitkatalysator kann als Pulver oder als verformter Katalysator in Form von Granulaten, Extrudaten oder Formkörpern eingesetzt werden. Zur Formgebung kann der Katalysator 1 bis 99 % eines Bindemittels oder Trägermaterials enthalten, wobei alle Bindemittel und Trägermaterialien geeignet sind, die unter den zur Epoxidierung angewandten Reaktionsbedingungen nicht mit Wasserstoffperoxid oder dem Epoxid reagieren. Bevorzugt werden Granulate entsprechend EP-A 0 893 158 oder Extrudate mit einem Durchmesser von 1 bis 5 mm eingesetzt.

Als Lösungsmittel geeignet sind alle Lösungsmittel, die unter den gewählten Reaktionsbedingungen nicht oder nur in geringem Maß durch Wasserstoffperoxid oxidiert werden und sich mit mehr als 10 Gew.-% in Wasser lösen. Bevorzugt werden Lösungsmittel, die mit Wasser unbegrenzt mischbar sind. Geeignete Lösungsmittel sind Alkohole wie z.B. Methanol, Ethanol oder tert-Butanol; Glykole wie z.B. Ethylenglykol, 1,2-Propandiol oder 1,3-Propandiol; cyclische Ether wie z.B. Tetrahydrofuran, Dioxan oder Propylenoxid; Glykolether wie z.B. Ethylenglykolmonomethylether, Ethylenglykolmonoethylether, Ethylenglykolmonobutylether oder die Propylenglykolmonomethylether und Ketone wie z.B. Aceton oder 2-Butanon. Bevorzugt wird Methanol als Lösungsmittel zugesetzt.

Als Basen können für das erfindungsgemäße Verfahren alle Stoffe eingesetzt werden, durch deren Zusatz der pH-Wert auf den erforderlichen Wert angehoben werden kann. Geeignete Basen sind Alkalimetallhydroxide, Ammoniak, Alkalimetallcarbonate, Ammoniumcarbonat, Alkalimetallhydrogencarbonate, Ammoniumhydrogencarbonat und Alkalimetall- und Ammoniumsalze von Carbonsäuren. Ebenfalls geeignet sind Alkalimetall- und Ammoniumsalze von mehrbasigen Mineralsäuren wie z.B. Phosphorsäure und Pyrophosphorsäure. Bevorzugt werden wässrige Lösungen der Base eingesetzt, besonders bevorzugt wässrige Lösungen von NaOH, LiOH oder Ammoniak. Gemäß einer weiteren Alternative handelt es sich bei der zur pH-Einstellung verwendeten Base um ein Puffergemisch, wie Borax/HCl, Borax/NaOH, NaH₂PO₄/NaOH.

Für die Einstellung des pH-Werts eignen sich alle physikalischen Größen und Meßverfahren, die einen vom pH-Wert in reproduzierbarer Weise abhängigen und sich mit dem pH-Wert verändernden Meßwert liefern. Bevorzugt wird eine potentiometrische Messung mit einer Glaselektrode eingesetzt, die ein pH-abhängiges Potential aufweist. Geeignet sind handelsübliche pH-Meßgeräte, bei denen das Potential direkt auf einer Skala in pH-Einheiten angezeigt wird und Elektroden, die bei den wässrigen Wasserstoffperoxidlösungen bzw. deren Mischungen mit dem Lösungsmittel ein stabiles und reproduzierbares Potential liefern. Durch den Gehalt an Wasserstoffperoxid und gegebenenfalls Lösungsmittel tritt an der Glaselektrode neben dem pH-abhängigen Potential zusätzlich ein Konzentrationspotential auf. Der mit einem handelsüblichen pH-Gerät gemessene pH-Wert weicht deshalb um einen konstanten Betrag vom tatsächlichen pH-Wert, d.h. dem dekadischen Logarithmus der Wasserstoffionenkonzentration, ab, wobei der Betrag in reproduzierbarer Weise vom Mischungsverhältnis von Wasser, Wasserstoffperoxid und gegebenenfalls Lösungsmittel abhängt. Für wässrige Wasserstoffperoxidlösungen ist diese Abweichung der pH-Messung mit einer Glaselektrode gegenüber dem tatsächlichen pH-Wert aus J. R. Kolczynski, E. M. Roth, E. S. Shanley, J. Am. Chem. Soc. 79 (1957) 531-533 bekannt.

Der für die Erzielung des erfindungsgemäßen Vorteils einzustellende pH-Wert hängt von der Zusammensetzung der Mischung aus Wässerstoffperoxid, Wasser und optional Lösungsmittel ab und läßt sich, wie die Beispiele belegen, in einfacher Weise durch eine Versuchsreihe bestimmen, bei der der pH-Wert variiert wird. Bei einer pH-Messung mit Glaselektrode wird der erfindungsgemäße Effekt bei Zugabe der Base zu einer wässrigen Wasserstoffperoxidlösung mit einem Gehalt zwischen 30 und 50 Gew.-% in der Regel erreicht, wenn der pH-Wert um 1 bis 4 pH-Einheiten angehoben wird und der vom Meßgerät angezeigte pH-Wert nach Zugabe der Base zwischen 4 und 6.5 liegt. In gleicher Weise wird der erfindungsgemäße Effekt bei Zugabe der Base zu einer Mischung aus wässrigem Wasserstoffperoxid und Methanol in der Regel erreicht, wenn der pH-Wert um 1 bis 6 pH-Einheiten angehoben wird und der vom Meßgerät angezeigte pH-Wert nach Zugabe der Base zwischen 5 und 9.5 liegt.

Das erfindungsgemäße Verfahren zur Epoxidierung von Propen wird bei einer Temperatur von -10 bis 100 °C, vorzugsweise bei 20 bis 70 °C durchgeführt. Das Propen wird vorzugsweise im Überschuß zu Wasserstoffperoxid eingesetzt um einen weitgehenden Wasserstoffperoxidumsatz zu erreichen, wobei das molare Verhältnis von Olefin zu Wasserstoffperoxid gleich/größer 1 ist und vorzugsweise im Bereich von 1.1 bis 10 liegt. Bei Zusatz eines organischen Lösungsmittels wird die Lösungsmittelmenge vorzugsweise so gewählt, daß in der Reaktionsmischung nur eine flüssige Phase vorliegt. Bevorzugt wird das Lösungsmittel in einem Gewichtsverhältnis von 1 bis 20 relativ zur eingesetzten Wasserstoffperoxidmenge zugesetzt.

Die eingesetzte Katalysatormenge kann in weiten Grenzen variiert werden und wird vorzugsweise so gewählt, dass unter den angewandten Reaktionsbedingungen innerhalb von 1 min bis 5 h ein Wasserstoffperoxidumsatz um 90 %, vorzugsweise mehr als 95 % erreicht wird.

Wird Propen umgesetzt, wobei dessen Siedepunkt bei Normaldruck unterhalb der gewählten Reaktionstemperatur liegt, dann wird die Reaktion bevorzugt unter Druck und unter einer Atmosphäre durchgeführt, die im wesentlichen aus dem dampfförmigem Olefin besteht; ein Olefin-Partialdruck im Bereich von 0.1 bis 1 MPa ist geeignet. Der Druck wird dabei besonders bevorzugt zwischen 50 und 100 % des Sättigungsdampfdrucks des Olefins bei der Reaktionstemperatur gewählt.

In einer Ausführungsform der Erfindung wird der Katalysator während der Epoxidierungsreaktion in der Reaktionsmischung suspendiert. Die Epoxidierungsreaktion kann in einem durchmischten Reaktor, z.B. einem Rührkessel oder einem Schlaufenreaktor durchgeführt werden.

In einer anderen bevorzugten Ausführungsform der Erfindung wird der Katalysator in Form einer Schüttung oder Packung in einem Rohrreaktor eingesetzt und die Mischung aus Wasserstoffperoxid, Olefin und optional Lösungsmittel über diese Packung geleitet. Bei unter den Reaktionsbedingungen gasförmigem Propen wird vorzugsweise zusätzlich noch gasförmiges Propen und gegebenenfalls zusätzlich ein inertes Gas eingespeist, wobei das gasförmige Propen oder Propen-Gas-Gemisch bevorzugt im Gegenstrom zur flüssigen Mischung geführt wird. Das gasförmige Propen oder Propen-Gas-Gemisch wird dabei bevorzugt von unten nach oben durch den Reaktor geführt, so dass es in Form von Blasen in der entgegenströmenden Lösung dispergiert wird. Die Menge des Gasstroms wird dabei so gewählt, dass am Ende des Reaktors nicht umgesetztes, gasförmiges Propen oder bei vollständigem Umsatz das Inertgas entnommen wird und mit diesem Gasstrom der durch Zersetzung von Wasserstoffperoxid im Reaktor gebildete molekulare Sauerstoff aus dem Reaktor ausgetragen wird.

Wenn der Katalysator in Form einer Schüttung oder Packung eingesetzt wird, kann er außerdem vor Beginn der Epoxidierungsreaktion konditioniert werden, indem Wasser, optional in Mischung mit einem Lösungsmittel und/oder Wasserstoffperoxid, durch Zusatz einer Base auf einen konstanten pH-Wert eingestellt und über den Katalysator geleitet wird.

Die zuvor genannte Blasenfahrweise eignet sich sowohl für die erfindungsgemäße Epoxidierung unter pH-Kontrolle als auch für andere gattungsgemäße Verfahren, etwa solchen, in welchen der Katalysator neutralisiert wird oder in welchen selektivitätsmindernde saure Funktionen des Katalysators durch chemische Umsetzung neutralisiert sind.

Das erfindungsgemäße Verfahren ermöglicht eine kontinuierliche Betriebsweise, ohne dass die Selektivität und Ausbeute (H₂O₂-Umsatz) durch Qualitätsschwankungen in den Einsatzstoffen negativ beeinflusst werden. Durch die erfindungsgemäße pH-kontrollierte separate Zudosierung einer Base während der Epoxidation erübrigt sich eine Behandlung des Katalysators vor oder während der Epoxidation.

### Beispiele

Für alle Beispiele wird als Katalysator ein Titansilikalit-Granulat verwendet, das nach dem in der EP 0 893 158, Beispiel 3 beschriebenen Verfahren hergestellt wurde. Die angegebenen Propylenoxidselektivitäten (PO-Selektivität) wurden als das Verhältnis der Konzentration von Propylenoxid zur Summe der Konzentrationen der Produkte Propylenoxid, 1-Methoxypropanol, 2-Methoxypropanol und 1,2-Propandiol berechnet.

### Beispiel 1 (Vergleichsbeispiel)

In einem thermostatisierten Laborautoklav mit Begasungsrührer werden 300 g Methanol bei 60 °C unter Propylenatmosphäre vorgelegt und bei 5 bar Überdruck mit Propylen gesättigt. Dann wird unter Rühren eine Mischung aus 518 g 50.7 Gew.-% Wasserstoffperoxid (destilliert), 2586 g Methanol, 125 g MTBE (tert-Butylmethylether), 253 g Wasser und 10 g Titansilicalit mit einer Rate von 290 g/h zudosiert. Gleichzeitig wird über ein Ventil so viel Reaktionsmischung entnommen, daß das Gewicht des Reaktorinhalts konstant blieb. Während der Dosierung wird über einen Druckregler Propylen nachdosiert, um den Druck im Reaktor konstant zu halten. In regelmäßigen Abständen wird in der entnommenen Reaktionsmischung der Wasserstoffperoxidgehalt durch Redoxtitration und der Gehalt an Propylenoxid, 1-Methoxy-2-propanol, 2-Methoxy-1-propanol und 1,2-Propandiol durch GC bestimmt. Nach 4 h wird ein stationärer Betriebszustand erreicht. Tabelle 1 zeigt den Wasserstoffperoxidumsatz und die Propylenoxidselektivität im stationären Betriebszustand.

Für das eingesetzte Wasserstoffperoxid wurde mit einer Einstabmeßkette aus Glaselektrode und integrierter Ag/AgCl-Vergleichselektrode ein pH-Wert von 2.8 gemessen. Korrigiert um das Konzentrationspotential für 50.7 Gew.-% Wasserstoffperoxid ergibt sich ein tatsächlicher pH-Wert von 4.6.

### Beispiele 2 bis 5

Beispiel 1 wird wiederholt, mit dem Unterschied, daß das eingesetzte Wasserstoffperoxid durch Zugabe von 1 N Natronlauge auf den in Tabelle 1 angeführten pH-Wert eingestellt wird, wobei der pH-Wert mit einer Einstabmeßkette aus Glaselektrode und integrierter Ag/AgCl-Vergleichselektrode gemessen wird. Nach 4.5 h wird ein stationärer Betriebszustand mit dem Wasserstoffperoxidumsatz und der Propylenoxidselektivität aus Tabelle 1 erreicht.

**Tabelle 1**

| Beispiel | pH des H₂O₂ | H₂O₂-Umsatz | PO-Selektivität |
|---|---|---|---|
| 1 | 2.8 | 71.0 % | 54.9 % |
| 2 | 4.0 | 71.4 % | 60.7 % |
| 3 | 4.5 | 69.5 % | 77.1 % |
| 4 | 4.75 | 65.3 % | 85.2 % |
| 5 | 5.0 | 35.3 % | 94.2 % |

### Beispiel 6 (Vergleichsbeispiel)

Beispiel 1 wird wiederholt, mit dem Unterschied, daß dem eingesetzten Wasserstoffperoxid soviel Natriumnitrat zugesetzt wird, daß es die gleiche Natriumkonzentration aufweist wie das in Beispiel 4 eingesetzt, mit Natronlauge auf pH 4.75 eingestellte Wasserstoffperoxid. Nach 4.5 h wird ein stationärer Betriebszustand mit einem Wasserstoffperoxidumsatz von 69.6 % und einer Propylenoxidselektivität von 68.0 % erreicht.

### Beispiel 7 (Vergleichsbeispiel)

Beispiel 1 wird wiederholt, mit dem Unterschied, daß ein anderer Titansilicalitkatalysator verwendet wird, die Reaktionstemperatur 65°C beträgt und eine Mischung aus 708 g 43.1 Gew.-% Wasserstoffperoxid (Rohprodukt aus dem Anthrachinonverfahren), 1743 g Methanol, 51 g MTBE und 35 g Titansilicalit mit einer Rate von 200 g/h zudosiert wird. Nach 4.5 h wird ein stationärer Betriebszustand erreicht. Tabelle 2 zeigt den Wasserstoffperoxidumsatz und die Propylenoxidselektivität im stationären Betriebszustand.

### Beispiele 8 bis 11

Beispiel 7 wird wiederholt, mit dem Unterschied, daß das eingesetzte Wasserstoffperoxid durch Zugabe von 25 Gew.-% wäßrigem Ammoniak auf den in Tabelle 2 angeführten pH-Wert eingestellt wird, wobei der pH-Wert mit einer Einstabmeßkette aus Glaselektrode und integrierter Ag/AgCl-Vergleichselektrode gemessen wird. Nach 4 bis 5.5 h wird ein stationärer Betriebszustand mit dem Wasserstoffperoxidumsatz und der Propylenoxidselektivität aus Tabelle 2 erreicht.

**Tabelle 2**

| Beispiel | pH des H₂O₂ | H₂O₂-Umsatz | PO-Selektivität |
|---|---|---|---|
| 7 | 2.42 | 58.5 % | 66.8 % |
| 8 | 5.15 | 53.2 % | 88.5 % |
| 9 | 5.35 | 50.5 % | 92.1 % |
| 10 | 5.55 | 41.2 % | 92.2 % |
| 11 | 5.75 | 27.1 % | 92.7 % |

### Beispiele 12 bis 15

Beispiel 7 wird wiederholt, mit dem Unterschied, daß die eingesetzte Mischung aus Wasserstoffperoxid, Methanol und MTBE vor Zugabe des Katalysators durch Zusatz von 25 Gew.-% wäßrigem Ammoniak auf den in Tabelle 3 angeführten pH-Wert eingestellt wird, wobei der pH-Wert mit einer Einstabmeßkette aus Glaselektrode und integrierter Ag/AgCl-Vergleichselektrode gemessen wird. Nach 4 bis 5.5 h wird ein stationärer Betriebszustand mit dem Wasserstoffperoxidumsatz und der Propylenoxidselektivität aus Tabelle 3 erreicht.

**Tabelle 3**

| Beispiel | pH der H₂O₂/MeOH-Mischung | H₂O₂-Umsatz | PO-Selektivität |
|---|---|---|---|
| 7 | 4.39 | 58.5 % | 66.8 % |
| 12 | 5.46 | 58.3 % | 78.4 % |
| 13 | 6.81 | 58.9 % | 80.6 % |
| 14 | 7.22 | 57.1 % | 81.7 % |
| 15 | 7.69 | 56.3 % | 86.7 % |
| 8 | 8.12 | 53.2 % | 88.5 % |
| 10 | 8.23 | 41.2 % | 92.2 % |
| 11 | 8.66 | 27.1 % | 92.7 % |

### Beispiele 16 bis 19

In einem thermostatisierten Rohrreaktor werden 65.7 g Titansilicalitkatalysator in Form von Extrudaten mit 2 mm Durchmesser vorgelegt. Eine Mischung aus 278 g 42.9 Gew.-% Wasserstoffperoxid, 6672 g Methanol und 51 g MTBE wird mit 25 Gew.-% wäßrigem Ammoniak auf den in Tabelle 4 angeführten pH-Wert eingestellt und anschließend bei 3 bar Überdruck und 45°C mit Propen gesättigt. Diese Mischung wird anschließend bei 39°C mit einer Rate von 900 g/h über den Katalysator geleitet. In regelmäßigen Abständen wird in der austretenden Reaktionsmischung der Wasserstoffperoxidgehalt durch Redoxtitration und der Gehalt an Propylenoxid, 1-Methoxy-2-propanol, 2-Methoxy-1-propanol und 1,2-Propandiol durch GC bestimmt. Nach 8 h Betrieb werden der in Tabelle 4 angeführte Wasserstoffperoxidumsatz und Propylenoxidselektivität erreicht.

**Tabelle 4**

| Beispiel | pH der H₂O₂/MeOH-Mischung | H₂O₂-Umsatz | PO-Selektivität |
|---|---|---|---|
| 16 | 5.5 | 94.9 % | 62.3 % |
| 17 | 8.5 | 74.3 % | 78.6 % |
| 18 | 8.7 | 65.9 % | 86.6 % |
| 19 | 8.9 | 56.1 % | 92.4 % |

### Beispiel 20 (Vergleichsbeispiel)

In einem thermostatisierten Rohrreaktor werden 68.0 g Titansilicalitkatalysator in Form von Extrudaten mit 2 mm Durchmesser vorgelegt. Bei 50 °C wird am unteren Ende des Reaktors eine Mischung aus 1334 g 42.9 Gew.-% Wasserstoffperoxid, 6600 g Methanol und 67 g MTBE mit einer Rate von 600 g/h eingespeist. Gleichzeitig werden am unteren Ende des Reaktors 200 g/h gasförmiges Propen zugegeben. Am oberen Ende des Reaktors wird die flüssige Reaktionsmischung und soviel nicht umgesetztes, gasförmiges Propen entnommen, daß ein Überdruck von 15 bar gehalten wird. Von der entnommenen Reaktionsmischung wird in regelmäßigen Abständen der Wasserstoffperoxidgehalt durch Redoxtitration und der Gehalt an Propylenoxid, 1-Methoxy-2-propanol, 2-Methoxy-1-propanol und 1,2-Propandiol durch GC bestimmt. Nach 8 h Betrieb wird der in Tabelle 5 angeführte Wasserstoffperoxidumsatz und Propylenoxidselektivität erreicht.

### Beispiele 21 bis 23

Beispiel 20 wird wiederholt, mit dem Unterschied, daß die eingesetzte Mischung aus Wasserstoffperoxid, Methanol und MTBE durch Zusatz von 25 Gew.-% wäßrigem Ammoniak auf den in Tabelle 5 angeführten pH-Wert eingestellt wird, wobei der pH-Wert mit einer Einstabmeßkette aus Glaselektrode und integrierter Ag/AgCl-Vergleichselektrode gemessen wird. Nach 8 h Betrieb werden der in Tabelle 5 angeführte Wasserstoffperoxidumsatz und Propylenoxidselektivität erreicht.

**Tabelle 5**

| Beispiel | pH der H₂O₂/MeOH-Mischung | H₂O₂-Umsatz | PO-Selektivität |
|---|---|---|---|
| 20 | 4.8 | 96.9 % | 81.7 % |
| 21 | 8.0 | 96.5 % | 87.2 % |
| 22 | 8.5 | 93.5 % | 94.8 % |
| 23 | 9.0 | 90.0 % | 94.6 % |

## Patentansprüche

1. Verfahren zur Herstellung von Propylenoxid umfassend Epoxidierung von Propen mit Wasserstoffperoxid in Gegenwart eines Titansilikalitkatalysators,
wobei eine Wasserstoffperoxid enthaltende Lösung und Propen als Einsatzstoffe periodisch oder kontinuierlich in einen Epoxidierungsreaktor eingetragen werden und im Verfahren zusätzlich eine Base eingesetzt wird,
**dadurch gekennzeichnet,**
**dass** man die Base unter pH-Kontrolle im Gemisch mit einem oder mehreren der Einsatzstoffe in den Epoxidierungsreaktor einträgt, die pH-Kontrolle in dem/den die Base enthaltenden Gemisch/en mit einem Einsatzstoff durchführt und einen pH-Wert im Bereich von 4 bis 9,5 einstellt und konstant hält.

2. Verfahren nach Anspruch 1,
**dadurch gekennzeichnet,**
**dass** man die Base einer wässrigen oder wässrig-organischen Wasserstoffperoxidlösung zusetzt und den pH-wert in der so erhaltenen Lösung einstellt und konstant hält, wobei im Falle der wässrigen Wasserstoffperoxidlösung der pH-Wert im Bereich von 4 bis 6.5 und im Falle einer organisch-wässrigen Wasserstoffperoxidlösung mit mindestens 50 Gew.-% eines organischen wasserlöslichen Lösungsmittels der pH-Wert im Bereich von 5 bis 9.5 liegt, wobei sich der pH-Wert auf eine Messung mittels einer Glaselektrode bezieht.

3. Verfahren nach Anspruch 1 oder 2,
**dadurch gekennzeichnet,**
**dass** man die Epoxidierung in Gegenwart eines organischen Lösungsmittels aus der Reihe niederer Alkohole oder/und Ether durchführt, wobei das Gewichtsverhältnis von Wasserstoffperoxid zu organischem Lösungsmittel im Bereich von 1 zu 1 bis 1 zu 20 liegt.

4. Verfahren nach einem der Ansprüche 1 bis 3,
**dadurch gekennzeichnet,**
**dass** man zur pH-Wert-Einstellung eine Base aus der Reihe der Alkalihydroxide, Alkalicarbonate, Alkalihydrogencarbonate, Alkaliphosphate. Alkalicarboxylate und Ammoniak verwendet, wobei die Base per se als wässrige Lösung eingesetzt wird.

5. Verfahren nach einem der Ansprüche 1 bis 4,
**dadurch gekennzeichnet,**
**dass** man die Epoxidierung in einem mit Titansilikalitkatalysator befüllten Festbettreaktor durchführt, wobei Propen und eine zwecks pH-Einstellung und Aufrechterhaltung zuvor mit einer Base versetz. te wässrige oder wässrig-organische Wasserstoffperoxidlösung im Gleich- oder Gegenstrom durch den Reaktor geleitet werden.

6. Verfahren nach einem der Ansprüche 1 bis 5,
**dadurch gekennzeichnet,**
**dass** man Propen bei einem Molverhältnis von Propen zu Wasserstoffperoxid im Bereich von gleich/größer 1 bis 10 epoxidiert, wobei die Epoxidierung in Gegenwart von Methanol bei einer Temperatur im Bereich von 20 bis 70°C und einem Propendruck im Bereich von 0.1 bis 1.0 MPa erfolgt.

7. Verfahren nach Anspruch 5 oder 6,
**dadurch gekennzeichnet,**
**dass** man ein unter Reaktionsbedingungen gasförmiges Propen epoxidiert, indem der Festbettreaktor als Blasensäule betrieben wird, wobei das Propen in der entgegenströmenden Lösung dispergiert wird.

## Claims

1. A process for the preparation of propylene oxide, comprising epoxidation of propene with hydrogen peroxide in the presence of a titanium silicalite catalyst,
whereby propene and a solution containing hydrogen peroxide are introduced as starting substances periodically or continuously into an epoxidation reactor, and, in addition, a base is used in the process,
**characterized in that**
the base is introduced with pH control into the epoxidation reactor as a mixture with at least one of the starting substances, pH control is carried out in the mixture(s) of the base with a starting substance, the pH is adjusted in a range of from 4 to 9.5 and is kept constant.

2. The process according to claim 1,
**characterized in that**
the base is added to an aqueous or aqueous-organic hydrogen peroxide solution, and the pH is adjusted and kept constant in the solution obtained, the pH of the aqueous hydrogen peroxide solution being in the range of from 4 to 6.5 and the pH of the aqueous-organic hydrogen peroxide solution with at least 50 weight percent of an organic water-soluble solvent being in the range of from 5 to 9.5, the pH being based on measurement by means of a glass electrode.

3. The process according to claim 1 or 2,
**characterized in that**
epoxidation is carried out in the presence of an organic solvent selected from the group consisting of lower alcohols or/and ethers, the weight ratio of hydrogen peroxide to organic solvent being in the range of from 1 : 1 to 1 : 20.

4. The process according to one of the claims 1 to 3,
**characterized in that**
a base selected from the group consisting of alkali metal hydroxides, alkali metal carbonates, alkali metal hydrogen carbonates, alkali metal phosphates, alkali metal carboxylates and ammonia is used to adjust the pH, the base being employed per se as an aqueous solution.

5. The process according to one of the claims 1 to 4,
**characterized in that**
the epoxidation is carried out in a fixed bed reactor filled with titanium silicalite catalyst, whereby propene and an aqueous or aqueous-organic solution of the hydrogen peroxide to which a base has been added beforehand for the purpose of adjusting and maintaining pH are passed in cocurrent flow or countercurrent flow through the reactor.

6. The process according to one of the claims 1 to 5,
**characterized in that**
propene is epoxidized at a molar ratio of propylene to hydrogen peroxide in the range of equal to or greater than 1 to 10, the epoxidation being carried out in the presence of methanol at a temperature in the range of from 20°C to 70°C under a propene pressure in the range of from 0.1 to 1.0 MPa.

7. The process according to claim 5 or 6,
**characterized in that**
a propene which is gaseous under the reaction conditions is epoxidized by operating the fixed bed reactor as a bubble column, the propene being dispersed in the solution by countercurrent flow.

## Revendications

1. Procédé de préparation d'oxyde de propylène, comprenant l'époxydation de propène avec du peroxyde d'hydrogène en présence d'un catalyseur constitué de silicalite au titane, selon lequel une solution contenant du peroxyde d'hydrogène ainsi que du propène sont introduits comme matières premières périodiquement ou en continu dans un réacteur d'époxydation, en utilisant également une base dans le procédé,
**caractérisé en ce que**
tout en contrôlant le pH, on introduit la base en mélange avec une ou plusieurs des matières premières dans le réacteur d'époxydation,
le contrôle du pH est effectué dans le(les) mélange(s) contenant la base et une matière première, on règle le pH et on le maintient à une valeur constante comprise dans une gamme allant de 4 à 9,5.

2. Procédé selon la revendication 1,
**caractérisé en ce que**
la base est ajoutée à une solution aqueuse ou organique aqueuse de peroxyde d'hydrogène, puis on règle le pH et on le maintient à une valeur constante dans la solution ainsi obtenue de sorte que, dans le cas de la solution aqueuse de peroxyde d'hydrogène, le pH se situe dans la gamme allant de 4 à 6,5 et, dans le cas d'une solution organique aqueuse de peroxyde d'hydrogène contenant au moins 50 % en poids d'un solvant organique hydrosoluble, le pH se situe dans la gamme allant de 5 à 9,5, le pH correspondant à une mesure effectuée au moyen d'une électrode en verre.

3. Procédé selon la revendication 1 ou 2,
**caractérisé en ce qu'**
on effectue l'époxydation en présence d'un solvant organique de la série constituée par les alcools inférieurs et/ou les éthers, le rapport en poids entre le peroxyde d'hydrogène et le solvant organique se situant dans la gamme allant de 1:1 à 1:20.

4. Procédé selon l'une des revendications 1 à 3,
**caractérisé en ce qu'**
pour le réglage du pH on utilise une base de la série constituée par les hydroxydes de métaux alcalins, les carbonates de métaux alcalins, les carbonates d'hydrogène de métaux alcalins, les phosphates de métaux alcalins, les carboxylates de métaux alcalins et l'ammoniac, la base étant, pour sa part, utilisée sous forme de solution aqueuse.

5. Procédé selon l'une des revendications 1 à 4,
**caractérisé en ce qu'**
on effectue l'époxydation dans un réacteur à lit fixe rempli d'un catalyseur constitué de silicalite au titane et l'on dirige du propène ainsi qu'une solution aqueuse ou organique aqueuse de peroxyde d'hydrogène, préalablement mélangée avec une base, dans le réacteur dans le même écoulement ou à contre-courant.

6. Procédé selon l'une des revendications 1 à 5,
**caractérisé en ce qu'**
on époxyde le propène à un rapport molaire entre propène et peroxyde d'hydrogène égal ou supérieur à 1 à 10, l'époxydation étant effectuée en présence de méthanol, à une température dans la gamme allant de 20 à 70°C et à une pression de propène comprise dans la gamme allant de 0,1 à 1,0 MPa.

7. Procédé selon la revendication 5 ou 6,
**caractérisé en ce qu'**
on époxyde un propène sous forme gazeuse dans les conditions de réaction, en faisant fonctionner le réacteur à lit fixe en tant que colonne à bulles et en dispersant le propène dans la solution s'écoulant en sens opposé.
